# EUROPEAN PATENT SPECIFICATION

(11) **EP 4 590 178 B1**
(45) Date of publication and mention of the grant of the patent: **25.02.2026**
(21) Application number: 23783523.6
(22) Date of filing: 18.09.2023
(51) Int. Cl.: A61B 1/018, A61N 1/05, A61B 5/00, A61B 1/313

(54) **DEVICE FOR NEUROPHYSIOLOGICAL ANALYSIS IN THE FIELD OF NEUROSURGICAL ENDOSCOPY AND ENDOSCOPIC GROUP INCLUDING THE DEVICE AND AN ENDOSCOPE**
VORRICHTUNG ZUR NEUROPHYSIOLOGISCHEN ANALYSE IM BEREICH DER NEUROCHIRURGISCHEN ENDOSKOPIE UND ENDOSKOPGRUPPE MIT DER VORRICHTUNG UND EINEM ENDOSKOP
DISPOSITIF D'ANALYSE NEUROPHYSIOLOGIQUE DANS LE DOMAINE DE L'ENDOSCOPIE NEUROCHIRURGICALE ET GROUPE ENDOSCOPIQUE COMPRENANT LE DISPOSITIF ET UN ENDOSCOPE

(30) Priority: 19.09.2022 IT 202200019119
(43) Date of publication of application: 30.07.2025
(73) Proprietor: Università Degli Studi Di Verona, 37129 Verona (IT)
(72) Inventor: FELETTI, Alberto, 37129 Verona (IT); SALA, Francesco, 37129 Verona (IT)
(74) Representative: Studio Torta S.p.A.
(86) International application number: PCT/IB2023/059220
(87) International publication number: WO 2024/062362

(56) References cited:
- US-A1- 2019 069 949

## Description

### Technical Field

The present invention relates to a device for neurophysiological analysis in the field of neurosurgical endoscopy and to an endoscopic group comprising the device and an endoscope. Specifically, the device comprises a sensitive element that is of the rigid or elastic type, is formed of a single-pole or two-pole electrode, and can be inserted into a work channel of the endoscope, which is also of the rigid or elastic type, respectively.

### Background

As is well known, intraoperative neurophysiology is currently a rapidly growing and developing field of research in neurosurgery because it allows, by recording and stimulating specific nerve structures of a patient, to guide the actions of a neurosurgeon by reducing the risks of functional damage to the examined nerve structures, and allows for some important prognostic assessments.

In a context wherein neurosurgery is increasingly reducing invasiveness while maintaining a high degree of surgical efficacy, neuroendoscopy has also been applied and progressively improved in recent years, thanks in part to significant technological advances, especially in the area of miniaturised intraoperative visualisation systems.

However, the state of the art still lacks the tools to allow neurophysiological recording and stimulation of ventricular surfaces during endoscopic neurosurgical interventions. This would allow the advantages that neurophysiology has made possible in open microsurgery to also be transferred to neuroendoscopy.

In particular, in recent years, brain surgery has mainly moved towards two goals: minimising the invasiveness of surgery and maximising patient safety.

In detail, endoscopic procedures have proven to be among the best strategies for reaching deep regions of the brain and for working on them by means of minimally invasive craniotomies, e.g. with drill holes even about 1 cm in diameter. This has been made possible by the technological development of endoscopes, which allow high-resolution vision through millimetre optical systems. This surgical procedure is particularly useful in brain ventricle surgery, which can be easily inspected using these technologies.

On the other hand, intra-operative neurophysiological mapping and monitoring (currently performed by removing a significant portion of the skull in order to uncover, and thus reach, the underlying brain region) make it possible to precisely localise the functional areas not only of the cerebral cortex, but also of the subcortical white matter, and to analyse neurological functions in real time during surgery, thus minimising the risk of neurological damage. However, to date there is no way of performing this mapping of the surface of the cerebral ventricles in a patient-safe and minimally invasive manner.

Document US 2019/069949 A1 relates generally to therapeutic tissue modulation and, more specifically, to embodiments of devices, systems and methods for therapeutically effecting neuromodulation of targeted nerve fibers of, for example, the hepatic system, to treat metabolic diseases or conditions, such as diabetes mellitus.

### Summary

The aim of the present invention is to provide a device for neurophysiological analysis in the field of neurosurgical endoscopy and an endoscopic group including the device and an endoscope, which overcome the drawbacks of the known art.

According to the present invention, a device for neurophysiological analysis in the field of neurosurgical endoscopy and an endoscopic group including the device and an endoscope, as defined in the appended claims, are provided.

### Brief Description of the Drawings

To better understand the present invention preferred embodiments thereof will be now described, for exemplary and non-limiting purposes, with reference to the appended claims, wherein:
- Figures 1 and 2 schematically show endoscopes of the known type, namely a rigid endoscope and an elastic endoscope, respectively;
- Figures 3 and 4 are schematic side views of respective embodiments of a device for neurophysiological analysis;
- Figures 5 and 6 are schematic cross-sectional views, along one of the cross-sectional lines A-A and B-B shown in Figures 3 and 4, respectively, of a sensitive element of the neurophysiological analysis device in Figures 3 or 4, according to their embodiments; and
- Figure 7 is a side view of an endoscope group comprising the endoscope of Figure 1 and the device for neurophysiological analysis of Figure 4.

In particular, the figures are shown with reference to a triaxial Cartesian system defined by an X axis, a Y axis and a Z axis, orthogonal to each other.

In the description below, elements common to the various embodiments of the invention are indicated with the same reference numbers.

### Description of Embodiments

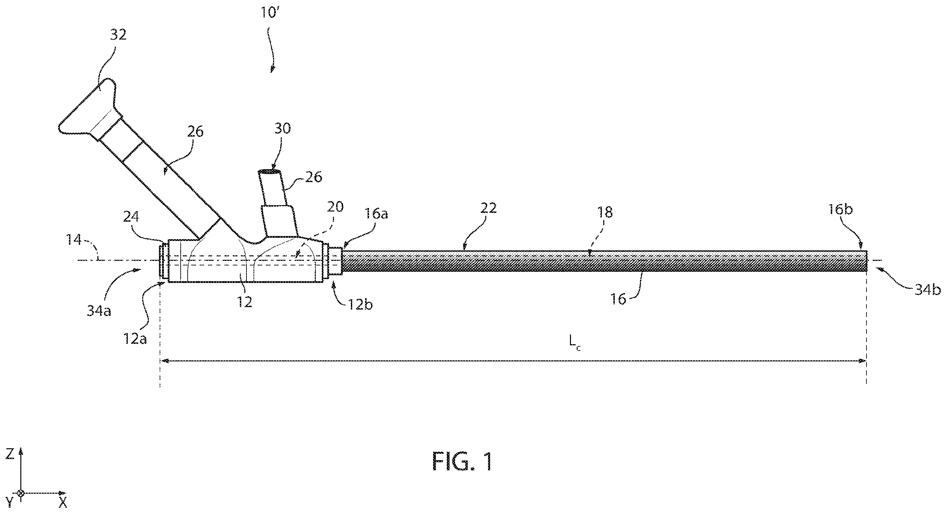

Figure 1 shows a known type of endoscope (examples may be those currently produced by the following companies:
Adeor Medical AG, Braun Melsungen AG, Clarus Medical, Karl Storz, Machida Endoscope, Codman, Medtronic, Olympus). In particular, it shows a rigid endoscope indicated in the figure by the reference number 10'.

For example, the rigid endoscope 10' can be used by a user (e.g., specialised medical personnel such as a neurosurgeon) on a patient during an endoscopic procedure, particularly in the field of neurosurgical endoscopy, to analyse or manipulate an anatomical region of interest of the patient (e.g., a region of the brain such as the lateral ventricle, the third ventricle, the sellar/parasellar region or the posterior cranial fossa).

The rigid endoscope 10' has a main body 12 of rigid type and having a first and a second end 12a and 12b opposite each other along a longitudinal axis 14 of the rigid endoscope 10', exemplified here parallel to the X axis.

A tubular element 16 of the rigid endoscope 10' is aligned with the main body 12 along the longitudinal axis 14 and has a proximal end 16a, coupled to the second end 12b of the main body 12, and a distal end 16b opposite the proximal end 16a along the longitudinal axis 14.

The tubular element 16 is of rigid type and defines a first channel 18 extending through the tubular element 16 along the longitudinal axis 14, from the proximal end 16a to the distal end 16b defining respective openings.

The main body 12 defines a second channel 20 extending through the main body 12 along the longitudinal axis 14, from the first to the second end 12a, 12b defining respective openings.

The first and second channels 18, 20 are aligned with each other along the longitudinal axis 14 with a break in continuity and with the opening of the proximal end 16a facing, and coinciding with, the opening of the second end 12b, so as to define a work channel 22 of the rigid endoscope 10' extending rectilinearly along the longitudinal axis 14, which communicates with the external environment through the openings of the first end 12a and the distal end 16b and into which surgical instruments can be inserted (not shown, e.g. forceps or endoscope scissors), in particular through the opening of the first end 12a. Accordingly, the openings of the first end 12a and the distal end 16b define, respectively, an inlet opening 34a and an outlet opening 34b of the work channel 22, through which the surgical instruments enter and exit the work channel 22, respectively.

Preferably, valve means 24 are included in the main body 12 and extend at the first opening 12a so as to allow the insertion of surgical instruments into the work channel 22 through the inlet opening 34a while preventing leakage of fluid from the work channel 22 through the inlet opening 34a.

Optionally and in a known way, the main body 12 may include additional elements useful during the endoscopic procedure. For example, it may comprise a tubular lighting element 26 defining a lighting channel (not shown) extending partly through the tubular lighting element 26 and partly through the main body 12 and the tubular lighting element 16, so as to reach the distal end 16b from a lighting opening 30 of the tubular lighting element 26 to which an external lighting device (not shown) is coupled in use that generates a light beam that, passing through the lighting channel, illuminates the anatomical region of interest at the distal end 16b. In addition, the main body 12 may for example also comprise a tubular viewing element 26 having an eyepiece 32 at one end thereof and defining a viewing channel (not shown) extending partly through the tubular viewing element 26 and partly through the main body 12 and the tubular viewing element 16 so as to allow visual inspection of the anatomical region of interest, for example by means of a set of lenses which allow, through the viewing channel, the image of the anatomical region of interest illuminated by the light beam to be transferred to the eyepiece 32.

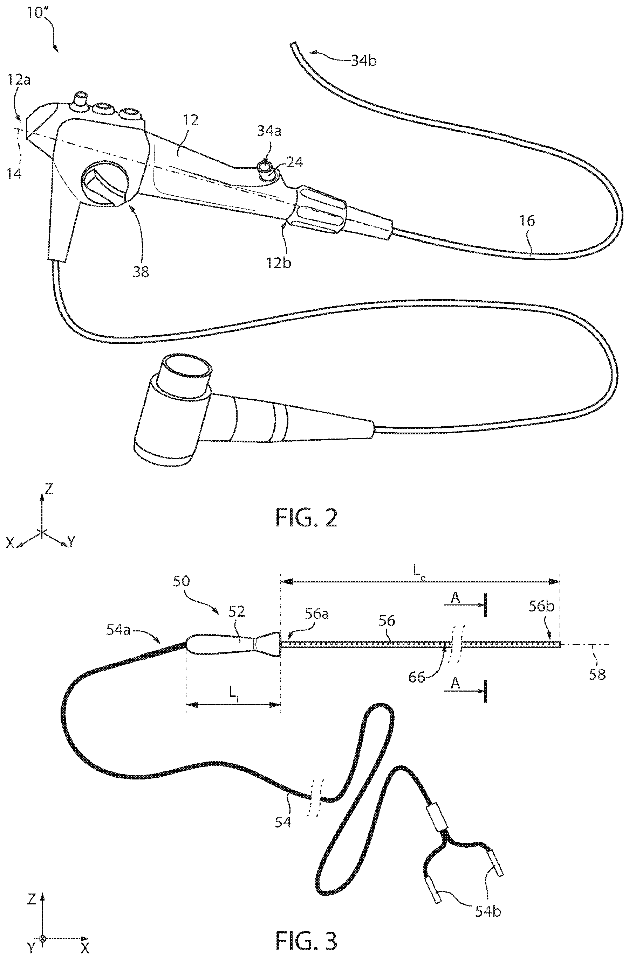

Figure 2 shows a different embodiment of a known type of endoscope, namely an elastic endoscope (also called a flexible endoscope), indicated in the figure with the reference number 10".

The elastic endoscope 10" is essentially analogous to the rigid endoscope 10', however, the tubular element 16 is elastic (in detail, to flexions) and deformable in a controlled manner by the user. Consequently, the work channel 22 may not extend in a rectilinear manner and be operated in such a way as to have a curved or serpentine shape, for example.

In particular, the tubular element 16 is controllable by means of actuating means 38 (e.g., push buttons or knobs included in the main body 12) operable to flex the tubular element 16. During the endoscopic procedure, the tubular element 16 is inserted into natural cavities or predetermined corridors of the patient in order to reach the anatomical region of interest; this is made possible by deforming the tubular element 16 in a controlled manner in order to follow these natural cavities or predetermined corridors of the patient to the anatomical region of interest. For example, in this case the lighting channel and the vision channel are realised using optical fibres so that the anatomical region of interest can be illuminated and visualised even when the tubular element 16 is bent.

Furthermore, the inlet opening 34a of the work channel 22 may not extend at the first end 12a and thus not be aligned with the distal end 16b along the longitudinal axis 14, but may for example extend into the main body 12 transverse to the longitudinal axis 14.

In general, therefore, the work channel 22 extends between the inlet opening 34a of the main body 12 and the outlet opening 34b of the tubular element 16, and in the rigid endoscope 10' the work channel 22 is rectilinear and the tubular element 16 is rigid while in the elastic endoscope 10" the work channel 22 may not be rectilinear and the tubular element 16 is elastic and may be bent and deformed.

Figure 3 shows a device for neurophysiological analysis 50 (hereafter also referred to more simply as device 50), according to an embodiment.

The device 50 comprises an electric connection element 54 (optional), a sensitive element 56 and a handgrip 52 that physically and electrically couples the electrical connection element 54 and the sensitive element 56 together.

In particular, the sensitive element 56 is of the single use type (i.e. disposable and is replaced after each endoscopic procedure so as to be used on only one patient and only once) and is coupled in a releasable manner with the handgrip 52 by means of known mechanical coupling techniques, thus it can be uncoupled from the handgrip and replaced with another sensitive element 56 after its use in the endoscopic procedure; i.e. it guarantees initial sterility and prevents contamination between different patients during endoscopic procedures. For example, the sensitive element 56 may be inserted, either by interlocking or by pressure, into a dedicated seat in the handgrip 52, or the handgrip 52 may comprise a clamp mechanism configured to close around part of the sensitive element to secure it to the handgrip 52.

In the exemplary embodiment shown in Figure 3, the sensitive element is a single-pole electrode (referred to below as a single-pole electrode and denoted by the reference 56'); however, as further described below, the sensitive element can also be a two-pole electrode (referred to below as a two-pole electrode and denoted by the reference 56").

In detail, the sensitive element 56 has an elongated shape, e.g. it is substantially threadlike.

The sensitive element 56 has a first end 56a and a second end 56b opposite each other along a main extension direction 58 of the electrode 56. The sensitive element 56 is coupled to the handgrip 52 through the first end 56a, which is configured to be coupled in a releasable manner with the seat of the handgrip 52.

The electric connection element 54 comprises an electric cable with a first end 54a and a second end 54b opposite each other. The first end 54a is coupled to the handgrip 52 while the second end 56b is electrically coupled to an external apparatus (of a known type and not part of the present invention) for polarising the sensitive element 56 and for acquiring the electrical signals detected through the sensitive element 56, actions which are of a known type and therefore not described in detail herein. For example, the second end 56b comprises a first and a second electrical terminal coupled to respective electric wires included in the electric connection element 54.

The handgrip 52 has a contoured shape to make it easier for the user to grip and handle the device 50.

For example, as shown by way of example in Figure 3, the handgrip 52 may have a shape elongated along the main extension direction 58 and tapered in the middle along the main extension direction 58 (i.e. the handgrip 52 has a section, transverse to the main extension direction 58, with an outer profile that narrows in a central portion and widens going from the central portion towards the end portions coupled to the sensitive element 56 and to the electric connection element 54). For example, in this case, the handgrip 52 may have a maximum handgrip length Lᵢ, measured in the main extension direction 58, comprised between approximately 3 cm and approximately 10 cm and, for example, approximately 4 cm.

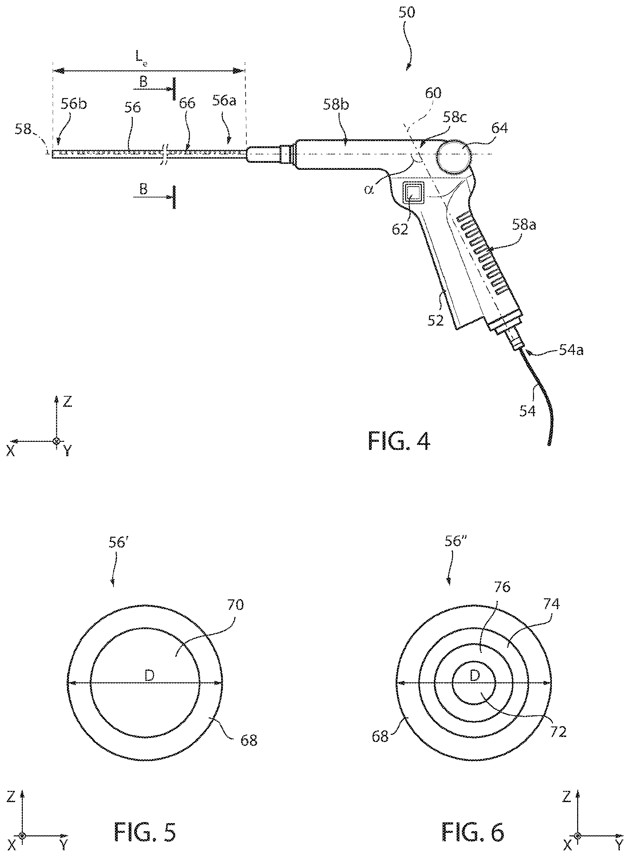

Otherwise, the handgrip 52 may, for example, have an "L" shape (in particular, a pistol shape) as shown in Figure 4. In other words, the handgrip 52 has a handgrip portion 58a and a support portion 58b joined together by a connection portion 58c which makes them monolithic to each other. The handgrip portion 58a extends primarily along a grip axis 60, transverse to the main extension direction 58, and is shaped to fit the hand of the operator and thus to facilitate the grip and manoeuvrability of the device 50 by the user, while the support portion 58b extends primarily along the main extension direction 58 and is coupled to the first end 56a of the sensitive element 56 to support the latter. In detail, the grip axis 60 and the main extension direction 58 form an angle α between them, at the connection portion 58c, which is preferably comprised between about 30° and about 100° to facilitate the manoeuvrability of the device 50. Indeed, it has been verified that this handgrip 52 prevents the user from manoeuvring the device 50 by essentially holding the arm parallel to the main extension direction 58, which would require the wrist to be held in an awkward position for the user for a prolonged time, causing discomfort and increasing the risk of errors when manoeuvring the device 50.

Furthermore, the handgrip 52 may comprise control means 62, 64 of the sensitive element 56 for electric control of the latter. In particular, the control means 62, 64 may comprise a push-button 62 and a knob 64, e.g. included in the connection portion 58c so as to be easily operable by the operator while gripping the handgrip portion 58a. The push-button 62 is actuated by the user to polarise or interrupt the polarisation of the sensitive element 56, while the knob 64 is rotated by the user about its axis of rotation in angular positions corresponding to respective values of electrical polarisation (electrical voltage) to be applied to the sensitive element 56. In this way, the user can control during endoscopic surgery not only the physical positioning of the sensitive element 56 but also its operation, polarising or not polarising the sensitive element 56 and choosing the level of polarisation to be applied. This is not possible in known solutions and the user has to communicate verbally with an additional operator to coordinate, the latter being in charge of controlling the electrical operation of the device 50 (e.g. through a PC usually placed at a distance from the patient).

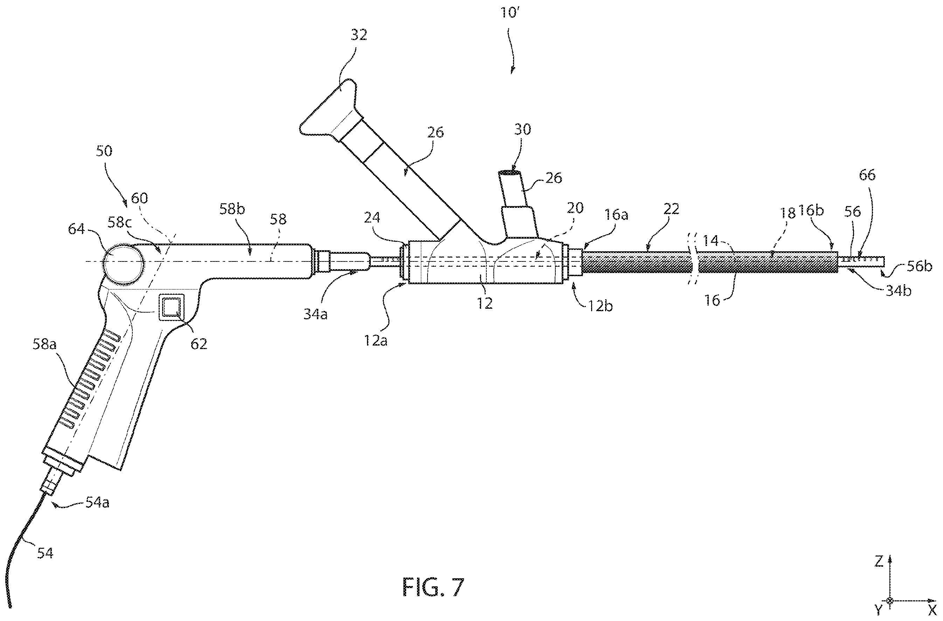

In use, the device 50 can be coupled to the endoscope 10', 10" to form an endoscope group (shown in Figure 7 with reference number 100, taking the rigid endoscope 10' as an example). In the following, reference is made to the case of the rigid endoscope 10', although the same discussion applies similarly to the case of the elastic endoscope 10".

Specifically, the device 50 is operated by the user so as to insert the sensitive element 56 into the work channel 22. In detail, the second end 56b of the sensitive element 56 is inserted through the inlet opening 34a so as to slide into the work channel 22 until it passes through the outlet opening 34b, in order to be in contact with the anatomical region of interest in order to stimulate it electrically and/or to detect its neurophysiological properties.

For this purpose, the sensitive element 56 has a first maximum length Lₑ between the first and second ends 56a and 56b (e.g. measured along the main extension direction 58) which is greater than a maximum length L_{c} of the work channel 22 measured (e.g. along the main extension direction 58, in the case of the rigid endoscope 10') between the inlet opening 34a and the outlet opening 34b.

Preferably, the sensitive element 56 has, on one of its outer surfaces, a graduated scale 66 that is progressive along the main extension direction 58. This allows the user to understand the position of the sensitive element 56 in the work channel 22 and thus the relative position of the second end 56b with respect to the anatomical region of interest. For example, the graduated scale 66 is provided, on the outer surface of the sensitive element 56, by a plurality of marks (optionally, numbered progressively), such as notches or lines orthogonal to the main extension direction 58. The marks may extend uniformly along the entire first maximum length Lₑ, or they may extend only at the first and second ends 56a and 56b (e.g., for a few cm from the first and second ends 56a and 56b towards the opposite end 56a and 56b), or they may present a step (i.e., a distance between signs consecutive to each other) that progressively decreases along the main extension direction 58 going from a central portion of the sensitive element 56 towards the first and/or second ends 56a, 56b, or they may present any combination of the options just described. In this way, the user can inspect the number of marks present between the first end 56a and the inlet opening 34a (e.g. visually) and/or the number of marks present between the second end 56b and the outlet opening 34b (e.g. by means of the eyepiece 32 or a camera connected to the endoscope 10', 10") and from this work out how far the second end 34b is from the anatomical region of interest.

In detail, the sensitive element 56 is formed by the single-pole electrode 56' or the two-pole electrode 56".

Figure 5 shows a cross-section (taken along one of the cross-section lines A-A and B-B shown in Figures 3 and 4 respectively and considered orthogonally to the main extension direction 58) of the sensitive element 56 formed through the single-pole electrode 56'. The single-pole electrode 56' comprises a first electrode 70 made of conductive material, in detail metal, surrounded externally by an outer insulating sheath 68 of the single-pole electrode 56'. The outer insulating sheath 68 is made of insulating material (e.g., Kapton, Silicone, Teflon and in detail PTFE-Teflon, HPDE, LDPE, PVDF, PVC), is concentric to the first electrode 70 and electrically isolates the first electrode 70 from the external environment so as to leave exposed axial end terminals which are opposite to each other along the main extension direction 58 and which form the first and second ends 56a and 56b of the single-pole electrode 56' (therefore in the following they may also be referred to as first and second ends 56a and 56b). In other words, the outer insulating sheath 68 surrounds a radial surface of the first electrode 70 and leaves the axial terminal ends of the first electrode 70 exposed. This is useful in the neurosurgical field as the anatomical region of interest to be examined is generally in an aqueous environment (e.g. the tubular element 16 is inserted into the patient's cerebral ventricles) and therefore the sensitive element 56 is generally in contact, during use, with body fluids which, in the absence of the outer insulating sheath 68, could interfere with the electrical measurement of the sensitive element 56. In this way, however, the first electrode 70 is electrically shielded from the surrounding aqueous environment, with the exception of the second electrode 56b placed at the anatomical region of interest to be examined or electrically stimulated. This allows the electrical interaction of the sensitive element 56 with the specific anatomical region of interest to be selective and localised.

In use, the single-pole electrode 56' is placed in contact with the anatomical region of interest through the endoscope 10', 10". The single-pole electrode 56' is operable in a stimulation operating condition wherein it is polarised by said external device, through the electric connection element 54, so as to electrically stimulate the anatomical region of interest (e.g., to assess the presence or absence of a response from the patient). Alternatively, the single-pole electrode 56' is operable in an acquisition operating condition wherein it acquires, through the single-pole electrode 56', electric signals indicative of an electrical action or neurophysiological state of the anatomical region of interest (e.g., related to diseases or damage of the anatomical region of interest).

Figure 6 instead shows a cross-section (taken along one of the cross-section lines A-A and B-B shown in Figures 3 and 4 respectively and considered orthogonally to the main extension direction 58) of the sensitive element 56 formed through the two-pole electrode 56". The two-pole electrode 56" comprises a first and a second electrode 72 and 74 made of conductive material, in detail metal. The first and second electrodes 72 and 74 are concentric with each other and are electrically insulated from each other by an intermediate insulating sheath 76 of the two-pole electrode 56", which is made of insulating material (e.g., Kapton, Silicone, Teflon and in detail PTFE-Teflon, HPDE, LDPE, PVDF, PVC), is concentric with the first and second electrodes 72 and 74 and is interposed between the first and second electrodes 72 and 74. For example, the second electrode 74 is external with respect to the first electrode 72. In addition, the two-pole electrode 56" may optionally also comprise the outer insulating sheath 68 surrounding the second electrode 74 and electrically insulating the first and second electrodes 72 and 74 from the external environment similarly to that previously discussed for the first electrode 70 (i.e., leaving the axial terminal ends of the first and second electrodes 72, 74 exposed at the second end 56b). In other words, the outer insulating sheath 68 surrounds a radial surface of the second electrode 74 and leaves the axial terminal ends of the first and second electrodes 72, 74 exposed.

In use, the two-pole electrode 56" is placed in contact with the anatomical region of interest through the endoscope 10', 10". In addition to the previously described operating conditions of stimulation and acquisition (wherein the first and second electrodes 72 and 74 form the anode and cathode of the two-pole electrode 56" and are polarised or, respectively, used to acquire electric signals from the anatomical region of interest), the two-pole electrode 56" is also operable in a mixed operating condition wherein one of the electrodes 72 and 74 (e.g., the first electrode 72) is polarised by said external apparatus so as to electrically stimulate the anatomical region of interest while the external apparatus acquires through the other electrode 74, 72 (in the example considered, the second electrode 74) the electrical signals indicative of the anatomical region of interest. In this way, stimulation and electrical detection are simultaneous.

Preferably, the sensitive element 56 has a circular profile in cross-section. Nevertheless, other closed polygonal shapes are possible, e.g. triangular, square or hexagonal.

The sensitive element 56 is designed to be elastic (in detail, to bending and, in greater detail, to flexions transverse to the main extension direction 58) or rigid depending on whether it is used in the elastic endoscope 10" or the rigid endoscope 10', respectively. In particular, in the case of use in the rigid endoscope 10' the first electrode 70 of the single-pole electrode 56' or the electrodes 72, 74 of the two-pole electrode 56" are made of rigid material such as stainless steel for surgical use (e.g., 304 Stainless Steel and 316 Stainless Steel), acrylonitrile butadiene styrene (ABS), acetal (Delrin), polyethylene terephthalate (PET) and polypropylene (PP), while in the case of use in the elastic endoscope 10" the first electrode 70 of the single-pole electrode 56' or the electrodes 72, 74 of the two-pole electrode 56" are made of elastic material such as HPDE, LDPE, PVDF Silicone, Teflon and in detail PTFE-Teflon, Kapton, PVC.

Furthermore, the sensitive element 56 has, orthogonally to the main extension direction 58, a maximum width D which is smaller than a minimum width of the work channel 22 (not shown and generally about 1.6 mm for the rigid endoscope 10' and about 1 mm for the elastic endoscope 10"). In this way, the sensitive element 56 can be inserted into the work channel 22. In particular, in the embodiments of Figures 5 and 6, the maximum width D is the diameter of the circular cross-section of the sensitive element 56 (e.g., outer diameter of the outer insulating sheath 68 or, in the case of the two-pole electrode 56" wherein the outer insulating sheath 68 is absent, outer diameter of the second electrode 74).

When the device 50 is used in the rigid endoscope 10', the maximum width D is comprised between about 0.1 mm and about 3 mm, in particular between about 0.5 mm and about 3 mm, and the first maximum length Lₑ is comprised between about 10 cm and about 70 cm, in particular between about 10 cm and about 40 cm. In contrast, when the device 50 is used in the elastic endoscope 10" the maximum width D is comprised between about 0.1 mm and about 3 mm, in particular between about 0.5 mm and about 3 mm, and the first maximum length Lₑ is comprised between about 15 cm and about 90 cm, in particular between about 15 mm and about 75 mm.

From an examination of the characteristics of the invention realised according to the present invention, the advantages that it allows obtaining are evident.

The device 50 can be used in conjunction with a rigid or elastic endoscope 10', 10" and can be used during endoscopic procedures, particularly for intraventricular neurosurgery.

Currently, there is no recording and/or stimulation device designed for use through the work channel 22 of a neurosurgical endoscope and for use on the ependymal surface in an aqueous environment such as the intraventricular one. The device 50 solves these problems, filling a gap in surgical and neurophysiological instrumentation.

Specifically, the device 50 allows the recording and stimulation of nerve structures in the intraventricular environment.

In contrast to known solutions that act by modulating peripheral nerve structures not in contact with the electrode but through the interposition of other tissues (e.g. the tunica adventitia and muscular layer of blood vessels), the sensitive element 56 of the device 50 is designed to come into direct contact with the central nerve tissue.

In addition, unlike known solutions wherein the electrode is inserted without any visual inspection, the device 50 can be coupled to the endoscope 10', 10" so that the sensitive element 56 can be inserted into the patient's body under direct endoscopic visualisation.

Furthermore, unlike known solutions wherein the electrodes are shaped so that the electrical contacts can take place on a tubular surface with an axis along the same axis as the electrodes, in the device 50 the sensitive element 56 can electrically contact the patient's body only at its tip (i.e. at the second end 56b) since the outer insulating sheath 68 electrically isolates, along the axis of the sensitive element 56, the remaining part of the sensitive element 56 from the patient's body in order to obtain a very selective measurement even on geometrically complex surfaces.

In addition, the sensitive element 56 is decoupled from the handgrip 52 and can therefore be easily replaced after each use. Since the sensitive element 56 is the only element of the device 50 that comes into direct contact with the patient's body, to ensure complete safety of the patient's health against cross-contamination between consecutive endoscopic procedures, it is sufficient to replace the disposable sensitive element 56 and sterilise the handgrip 52. Accordingly, the device 50 is a class III medical product that nevertheless presents a reduced overall cost (as the handgrip 52 and the electrical connection element 54 are autoclavable and can be reused without needing to be replaced each time the device 50 is used).

In general, the device 50 reduces the invasive impact of interventions on the patient by allowing endoscopic procedures to also be used for neurosurgical analysis and neurophysiological monitoring.

It is clear that changes and variations can be made to the invention described and shown herein without departing from the protection scope of the present invention, as defined in the appended claims.

For example, the different embodiments described can be combined to provide further solutions.

## Claims

1. A device (50) for neurophysiological analysis of an anatomical region of interest of a patient in the field of neurosurgical endoscopy, the device (50) comprising:
- a handgrip (52);
- a sensitive element (56) having a first (56a) and a second end (56b) opposite each other in a main extension direction (58) of the sensitive element (56), the sensitive element (56) being coupled in a releasable manner with the handgrip (52) through the first end (56a),
wherein the sensitive element (56) is formed of a single-pole electrode (56') or a two-pole electrode (56"), and
wherein the sensitive element (56) is of rigid type and has a maximum width (D), measured orthogonally to the main extension direction (58), ranging from 0.1 mm to 3 mm and a first maximum length (Lₑ), measured in the main extension direction (58), ranging from 10 cm to 70 cm, or is of elastic type to flexions transverse to the main extension direction (58) and has the maximum width (D) ranging from 0.1 mm to 3 mm and the first maximum length (Lₑ) ranging from 15 cm to 90 cm,
wherein the sensitive element (56) is formed by said single-pole electrode (56') that includes a first electrode (70), made of conductive material, and an outer insulating sheath (68), made of insulating material, that surrounds a radial surface of the first electrode (70) leaving exposed axial terminal ends of the first electrode (70), or
wherein the sensitive element (56) is formed by said two-pole electrode (56") which includes a first electrode (72) and a second electrode (74) made of conductive material and concentric to each other in the main extension direction (58), and an intermediate insulating sheath (76) interposed between the first electrode (72) and the second electrode (74) to electrically insulate from each other the first electrode (72) and the second electrode (74), the second electrode (74) being external to the first electrode (72), and
wherein, if the sensitive element (56) is formed by said two-pole electrode (56"), the two-pole electrode (56") further includes an outer insulating sheath (68), made of insulating material, which surrounds a radial surface of the second electrode (74) leaving exposed axial terminal ends of the first and second electrode (72, 74).

2. The device according to claim 1, wherein the sensitive element (56) has a shape elongated in the main extension direction (58).

3. The device according to claim 1 or 2, wherein the sensitive element (56) has an outer surface provided with a graduated scale that is progressive in the main extension direction (58).

4. The device according to any one of the preceding claims, wherein the handgrip (52) has a shape tapered in the centre along the main extension direction (58), or has an L shape.

5. The device according to any one of the preceding claims, wherein the handgrip (52) comprises control means (62, 64) for electric control of the sensitive element (56).

6. The device according to claim 5, wherein the control means (62, 64) comprise a push-button (62) to activate and de-activate an electric polarisation of the sensitive element (56), and a knob (64) for selecting the level of said electric polarisation of the sensitive element (56).

7. The device according to any one of the preceding claims, further comprising an electric connection element (54) which has a respective first end (54a) and a respective second end (54b) opposite to each other, is physically coupled to the handgrip (52) through the first end (54a) of the electric connection element (54), is electrically coupled to the sensitive element (56) and is electrically couplable, through the second end (54b) of the electric connection element (54), to an external apparatus,
the sensitive element (56) being electrically polarisable by the external apparatus by means of the electric connection element (54).

8. The device according to any one of the preceding claims, wherein the sensitive element (56) is of the disposable type.

9. An endoscopic group (100) comprising a device (50) according to any one of the claims 1-8 and an endoscope (10'; 10"),
wherein the endoscope (10'; 10") comprises a main body (12) of rigid type and a tubular element (16) of rigid type or of elastic type to flexions transverse to the main extension direction (58), fixed to one another and defining a work channel (18) that extends between an inlet opening (34a) of the main body (12) and an outlet opening (34b) of the tubular element (16), the outlet opening (34b) extending at a distal end (16b) of the tubular element (16) opposite the main body (12) along the main extension direction (58),
wherein the device (50) is couplable to the endoscope (10'; 10") so that the sensitive element (56) extends into the work channel (18), from the inlet opening (34a) and until the second end (56b) of the sensitive element (56) passes through the outlet opening (34b), and
wherein the sensitive element (56) of the device (50) is rigid if the tubular element (16) of the endoscope (10') is rigid and the sensitive element (56) of the device (50) is elastic if the tubular element (16) of the endoscope (10') is elastic.

## Patentansprüche

1. Vorrichtung (50) zur neurophysiologischen Analyse einer anatomischen Region von Interesse eines Patienten auf dem Gebiet der neurochirurgischen Endoskopie, wobei die Vorrichtung (50) umfasst:
- einen Handgriff (52);
- ein empfindliches Element (56) mit einem ersten (56a) und einem zweiten Ende (56b), die einander in einer Haupterstreckungsrichtung (58) des empfindlichen Elements (56) gegenüberliegen, wobei das empfindliche Element (56) über das erste Ende (56a) lösbar mit dem Handgriff (52) gekoppelt ist,
wobei das empfindliche Element (56) aus einer einpoligen Elektrode (56') oder einer zweipoligen Elektrode (56") gebildet ist, und
wobei das empfindliche Element (56) vom starren Typ ist und eine maximale Breite (D), gemessen orthogonal zur Haupterstreckungsrichtung (58), im Bereich von 0,1 mm bis 3 mm und eine erste maximale Länge (Le), gemessen in der Haupterstreckungsrichtung (58), im Bereich von 10 cm bis 70 cm aufweist, oder vom elastischen Typ für Biegungen quer zur Haupterstreckungsrichtung (58) ist, und die maximale Breite (D) im Bereich von 0,1 mm bis 3 mm und die erste maximale Länge (Le) im Bereich von 15 cm bis 90 cm aufweist,
wobei das empfindliche Element (56) durch die einpolige Elektrode (56') gebildet wird, die eine erste Elektrode (70) aus leitendem Material und eine äußere isolierende Hülle (68) aus isolierendem Material beinhaltet, die eine radiale Oberfläche der ersten Elektrode (70) umgibt und axiale Anschlussenden der ersten Elektrode (70) freiliegend lässt, oder
wobei das empfindliche Element (56) durch die zweipolige Elektrode (56") gebildet wird, die eine erste Elektrode (72) und eine zweite Elektrode (74), die aus leitendem Material bestehen und in der Haupterstreckungsrichtung (58) konzentrisch zueinander sind, und eine isolierende Zwischenhülle (76) beinhaltet, die zwischen der ersten Elektrode (72) und der zweiten Elektrode (74) angeordnet ist, um die erste Elektrode (72) und die zweite Elektrode (74) elektrisch voneinander zu isolieren, wobei sich die zweite Elektrode (74) außerhalb der ersten Elektrode (72) befindet, und
wobei, wenn das empfindliche Element (56) durch die zweipolige Elektrode (56") gebildet wird, die zweipolige Elektrode (56") ferner eine äußere isolierende Hülle (68) aus isolierendem Material beinhaltet, die eine radiale Oberfläche der zweiten Elektrode (74) umgibt und axiale Anschlussenden der ersten und zweiten Elektrode (72, 74) freiliegend lässt.

2. Vorrichtung nach Anspruch 1, wobei das empfindliche Element (56) eine in der Haupterstreckungsrichtung (58) langgestreckte Form aufweist.

3. Vorrichtung nach Anspruch 1 oder 2, wobei das empfindliche Element (56) eine äußere Oberfläche aufweist, die mit einer Messskala versehen ist, die in der Haupterstreckungsrichtung (58) progressiv ist.

4. Vorrichtung nach einem der vorhergehenden Ansprüche, wobei der Handgriff (52) eine Form aufweist, die sich in der Mitte entlang der Haupterstreckungsrichtung (58) verjüngt, oder eine L-Form aufweist.

5. Vorrichtung nach einem der vorhergehenden Ansprüche, wobei der Handgriff (52) Steuermittel (62, 64) zur elektrischen Steuerung des empfindlichen Elements (56) umfasst.

6. Vorrichtung nach Anspruch 5, wobei die Steuermittel (62, 64) einen Druckknopf (62) zum Aktivieren und Deaktivieren einer elektrischen Polarisation des empfindlichen Elements (56) und einen Drehknopf (64) zum Auswählen des Niveaus der elektrischen Polarisation des empfindlichen Elements (56) umfassen.

7. Vorrichtung nach einem der vorhergehenden Ansprüche, ferner umfassend ein elektrisches Verbindungselement (54), das ein jeweiliges erstes Ende (54a) und ein jeweiliges zweites Ende (54b) aufweist, die einander gegenüberliegen, physisch mit dem Handgriff (52) durch das erste Ende (54a) des elektrischen Verbindungselements (54) gekoppelt ist, elektrisch mit dem empfindlichen Element (56) gekoppelt ist und elektrisch durch das zweite Ende (54b) des elektrischen Verbindungselements (54) mit einem externen Gerät koppelbar ist,
wobei das empfindliche Element (56) durch das externe Gerät mittels des elektrischen Verbindungselements (54) elektrisch polarisierbar ist.

8. Vorrichtung nach einem der vorhergehenden Ansprüche, wobei das empfindliche Element (56) vom Einwegtyp ist.

9. Endoskopische Gruppe (100), umfassend eine Vorrichtung (50) nach einem der Ansprüche 1-8 und ein Endoskop (10'; 10"),
wobei das Endoskop (10'; 10") einen Hauptkörper (12) vom starren Typ und ein rohrförmiges Element (16) vom starren Typ oder vom elastischen Typ für Biegungen quer zur Haupterstreckungsrichtung (58) umfasst, die aneinander befestigt sind und einen Arbeitskanal (18) definieren, der sich zwischen einer Einlassöffnung (34a) des Hauptkörpers (12) und einer Auslassöffnung (34b) des rohrförmigen Elements (16) erstreckt, wobei sich die Auslassöffnung (34b) an einem distalen Ende (16b) des rohrförmigen Elements (16) gegenüber dem Hauptkörper (12) entlang der Haupterstreckungsrichtung (58) erstreckt, wobei die Vorrichtung (50) mit dem Endoskop (10'; 10") koppelbar ist, so dass sich das empfindliche Element (56) von der Einlassöffnung (34a) in den Arbeitskanal (18) erstreckt und bis das zweite Ende (56b) des empfindlichen Elements (56) durch die Auslassöffnung (34b) verläuft, und
wobei das empfindliche Element (56) der Vorrichtung (50) starr ist, wenn das rohrförmige Element (16) des Endoskops (10') starr ist, und das empfindliche Element (56) der Vorrichtung (50) elastisch ist, wenn das rohrförmige Element (16) des Endoskops (10') elastisch ist.

## Revendications

1. Dispositif (50) d'analyse neurophysiologique d'une région anatomique d'intérêt d'un patient dans le domaine de l'endoscopie neurochirurgicale, le dispositif (50) comprenant :
- une poignée (52) ;
- un élément sensible (56) comportant une première (56a) et une seconde extrémité (56b) opposées l'une à l'autre dans une direction d'extension principale (58) de l'élément sensible (56), l'élément sensible (56) étant accouplé de manière amovible à la poignée (52) par la première extrémité (56a),
l'élément sensible (56) étant formé d'une électrode unipolaire (56') ou d'une électrode bipolaire (56"), et
l'élément sensible (56) étant de type rigide et ayant une largeur maximale (D), mesurée orthogonalement à la direction d'extension principale (58), allant de 0,1 à 3 mm et une première longueur maximale (Le), mesurée dans la direction d'extension principale (58), allant de 10 à 70 cm, ou étant de type élastique à flexions transversales à la direction d'extension principale (58) et ayant la largeur maximale (D) allant de 0,1 à 3 mm et la première longueur maximale (Le) allant de 15 à 90 cm,
l'élément sensible (56) étant formé par ladite électrode unipolaire (56') qui comprend une première électrode (70), constituée d'un matériau conducteur, et une gaine isolante extérieure (68), constituée d'un matériau isolant, qui entoure une surface radiale de la première électrode (70) en laissant exposées les extrémités axiales de borne de la première électrode (70), ou
l'élément sensible (56) étant formé par ladite électrode bipolaire (56") qui comprend une première électrode (72) et une seconde électrode (74) constituées d'un matériau conducteur et concentriques l'une par rapport à l'autre dans la direction d'extension principale (58), et une gaine isolante intermédiaire (76) interposée entre la première électrode (72) et la seconde électrode (74) pour isoler électriquement l'une de l'autre la première électrode (72) et la seconde électrode (74), la seconde électrode (74) étant extérieure à la première électrode (72), et
où, si l'élément sensible (56) est formé par ladite électrode bipolaire (56"), l'électrode bipolaire (56") comprend en outre une gaine isolante extérieure (68), constituée d'un matériau isolant, qui entoure une surface radiale de la seconde électrode (74) en laissant exposées les extrémités axiales de borne de la première et de la seconde électrode (72, 74).

2. Dispositif selon la revendication 1, l'élément sensible (56) ayant une forme allongée dans la direction d'extension principale (58).

3. Dispositif selon la revendication 1 ou 2, l'élément sensible (56) présentant une surface extérieure pourvue d'une échelle graduée qui est progressive dans la direction d'extension principale (58).

4. Dispositif selon l'une quelconque des revendications précédentes, la poignée (52) ayant une forme effilée au centre le long de la direction d'extension principale (58), ou ayant une forme de L.

5. Dispositif selon l'une quelconque des revendications précédentes, la poignée (52) comprenant des moyens de commande (62, 64) pour la commande électrique de l'élément sensible (56).

6. Dispositif selon la revendication 5, les moyens de commande (62, 64) comprenant un boutonpoussoir (62) pour activer et désactiver une polarisation électrique de l'élément sensible (56), et un bouton (64) pour sélectionner le niveau de ladite polarisation électrique de l'élément sensible (56).

7. Dispositif selon l'une quelconque des revendications précédentes, comprenant en outre un élément de raccordement électrique (54), qui comporte une première extrémité respective (54a) et une seconde extrémité respective (54b) opposées l'une à l'autre, est physiquement accouplé à la poignée (52) par la première extrémité (54a) de l'élément de raccordement électrique (54), est électriquement accouplé à l'élément sensible (56) et pouvant être électriquement accouplé, par la seconde extrémité (54b) de l'élément de raccordement électrique (54), à un appareil externe,
l'élément sensible (56) étant polarisable électriquement par l'appareil externe au moyen de l'élément de raccordement électrique (54).

8. Dispositif selon l'une quelconque des revendications précédentes, l'élément sensible (56) étant du type jetable.

9. Groupe endoscopique (100) comprenant un dispositif (50) selon l'une quelconque des revendications 1-8 et un endoscope (10' ; 10"),
l'endoscope (10' ; 10") comprenant un corps principal (12) de type rigide et un élément tubulaire (16) de type rigide ou de type élastique à flexions transversales à la direction d'extension principale (58), fixés l'un à l'autre et définissant un canal de travail (18) qui s'étend entre une ouverture d'entrée (34a) du corps principal (12) et une ouverture de sortie (34b) de l'élément tubulaire (16), l'ouverture de sortie (34b) s'étendant à une extrémité distale (16b) de l'élément tubulaire (16) opposée au corps principal (12) le long de la direction d'extension principale (58), le dispositif (50) pouvant être accouplé à l'endoscope (10' ; 10") de manière à ce que l'élément sensible (56) s'étende dans le canal de travail (18), à partir de l'ouverture d'entrée (34a) et jusqu'à ce que la seconde extrémité (56b) de l'élément sensible (56) passe à travers l'ouverture de sortie (34b), et
l'élément sensible (56) du dispositif (50) étant rigide si l'élément tubulaire (16) de l'endoscope (10') est rigide et l'élément sensible (56) du dispositif (50) étant élastique si l'élément tubulaire (16) de l'endoscope (10') est élastique.
